# EUROPEAN PATENT APPLICATION

(11) **EP 3 821 823 A1**
(43) Date of publication of application: **19.05.2021**
(21) Application number: 18925787.6
(22) Date of filing: 11.07.2018
(51) Int. Cl.: A61B 17/12

(54) **LIGATION DEVICE**

(71) Applicant: ASAHI INTECC CO., LTD., Seto-shi, Aichi 489-0071 (JP)
(72) Inventor: KIKUCHI, Daisuke, Tokyo 152-0021 (JP); TSUKAMOTO, Toshihiko, Seto-shi, Aichi 489-0071 (JP); NAMIMA, Satoshi, Seto-shi, Aichi 489-0071 (JP); ASAHATA, Erika, Seto-shi, Aichi 489-0071 (JP); UTANI, Takayuki, Seto-shi, Aichi 489-0071 (JP)
(74) Representative: Winter, Brandl - Partnerschaft mbB
(86) International application number: PCT/JP2018/026228
(87) International publication number: WO 2020/012585

(57) **Abstract**

A ligation device capable of reliably ejecting a ligation ring from a main body is provided. A ligation device 1 includes a main body 10 having a cylindrical shape, located on an endoscope distal end portion 2b, having a distal end portion 11 projecting toward a distal end side of a distal end face 2c of an endoscope 2, and having a first fluid feeding passage 12 with a first opening 14 that opens on a distal end face 13 of the distal end portion 11; and a first ligation ring 5 attached to the distal end portion 11 of the main body 10 so as to obstruct the first opening 14.

## Description

### TECHNICAL FIELD

The present invention relates to a ligation device.

### BACKGROUND ART

A ligation device for ligating an affected part such as a diverticula and a varix formed in a patient's digestive tract or the like is known. Such a ligation device is disclosed in e.g. PTL1. With a ligation kit disclosed in PTL1, a slide cylinder is driven by a fluid, and an O-ring attached on an inner cylinder is pushed out and ejected from the inner cylinder by the slide cylinder to ligate an affected part.

### CITATION LIST

### Patent Literature

PTL1: Japanese Patent Laid-Open No. 7-59786

### SUMMARY OF INVENTION

### Technical Problem

However, with the ligation kit in PTL1, the O-ring stretched and attached to the inner cylinder is pushed out by the fluid-driven slide cylinder, to eject the O-ring from the inner cylinder. Thus, a force for ejecting the O-ring has been weak, and it has been impossible to successfully eject the O-ring.

An object of the present invention is to provide a ligation device capable of reliably ejecting the ligation ring from the main body.

### Solution to Problem

In order to achieve such an object, the ligation device according to an aspect of the present invention includes a main body having a cylindrical shape, located on an endoscope distal end portion, having a distal end portion projecting toward a distal end side of a distal end face of the endoscope, and having a first fluid feeding passage with a first opening that opens on a distal end face or an outer peripheral face of the distal end portion; and a first ligation ring attached to the distal end portion of the main body so as to obstruct the first opening.

The main body may have a second fluid feeding passage with a second opening that opens on the distal end face or the outer peripheral face of the distal end portion, and a second ligation ring may be attached to the distal end portion of the main body so as to obstruct the second opening of the second fluid feeding passage.

The distal end portion of the main body may have a tapered shape that tapers toward the distal end, and the first opening of the first fluid feeding passage and the second opening of the second fluid feeding passage may open on the outer peripheral face of the distal end portion of the main body.

The main body may have a cylindrical base portion and a cylindrical sliding portion placed so as to be slidable with respect to the base portion by a fluid, the first fluid feeding passage is formed on the sliding portion, the first opening may open on a distal end face or an outer peripheral face of the sliding portion, and the first ligation ring may be attached to the sliding portion.

The distal end portion of the base portion may have a tapered shape that tapers toward the distal end.

The sliding portion may have the second fluid feeding passage having the second opening that opens on the distal end face or the outer peripheral face of the sliding portion, and the second ligation ring may be attached to the sliding portion so as to obstruct the second opening of the second fluid feeding passage.

A distal end portion of the sliding portion may have a tapered shape that tapers toward the distal end, and the first opening of the first fluid feeding passage and the second opening of the second fluid feeding passage may open on an outer peripheral face of the distal end portion of the sliding portion.

The first opening may have an annular shape coaxial with the cylindrical main body, the first ligation ring may be placed on the main body so as to obstruct the first annular opening, and the second ligation ring may be placed on a proximal end side of the first opening with respect to the first ligation ring.

The main body may have a cylindrical base portion and a cylindrical sliding portion placed so as to be slidable with respect to the base portion by a fluid, the first fluid feeding passage is formed on the sliding portion, the first opening opens on the distal end face of the sliding portion, and the first opening may have an annular shape coaxial with the cylindrical sliding portion.

The distal end portion of the base portion may have a tapered shape that tapers toward the distal end.

A projection on which a distal end of the endoscope distal end portion abuts may be placed on an inner peripheral face of the main body.

The main body may be made of a translucent material. Advantageous Effect of Invention

The present invention can provide a ligation device capable of reliably ejecting the ligation ring from the main body.

### BRIEF DESCRIPTION OF DRAWINGS

FIG. 1 (a) is a sectional view of a ligation device according to the first embodiment attached to an endoscope.
FIG. 1 (b) is a front view of the ligation device according to the first embodiment attached to the endoscope.
FIG. 2 (a) is a sectional view of a ligation device according to the second embodiment attached to an endoscope.
FIG. 2 (b) is a front view of the ligation device according to the second embodiment attached to the endoscope.
FIG. 3 (a) is a sectional view of a ligation device according to the third embodiment attached to an endoscope.
FIG. 3 (b) is a front view of the ligation device according to the third embodiment attached to the endoscope.
FIG. 4 (a) is a sectional view of a ligation device according to the fourth embodiment attached to an endoscope.
FIG. 4 (b) is a front view of the ligation device according to the fourth embodiment attached to the endoscope.
FIG. 5 (a) is a sectional view of a ligation device according to the fifth embodiment attached to an endoscope.
FIG. 5 (b) is a front view of the ligation device according to the fifth embodiment attached to the endoscope.
FIG. 6 (a) is a sectional view of a ligation device according to the sixth embodiment attached to an endoscope.
FIG. 6 (b) is a front view of the ligation device according to the sixth embodiment attached to the endoscope.
FIG. 7 (a) is a sectional view of a ligation device according to the seventh embodiment attached to an endoscope, illustrating a state that a sliding portion is located on a proximal end side.
FIG. 7 (b) is a sectional view of the ligation device according to the seventh embodiment attached to the endoscope, illustrating a state that the sliding portion is located on a distal end side.
FIG. 8 (a) is a sectional view of a ligation device according to the eighth embodiment attached to an endoscope, illustrating a state that a sliding portion is located on a proximal end side.
FIG. 8 (b) is a sectional view of the ligation device according to the eighth embodiment attached to the endoscope, illustrating a state that the sliding portion is located on a distal end side.
FIG. 9 (a) is a sectional view of a ligation device according to the ninth embodiment attached to an endoscope, illustrating a state that a sliding portion is located on a proximal end side.
FIG. 9 (b) is a sectional view of the ligation device according to the ninth embodiment attached to the endoscope, illustrating a state that the sliding portion is located on a distal end side.
FIG. 10 (a) is a sectional view of a ligation device according to the tenth embodiment attached to an endoscope, illustrating a state that a sliding portion is located on a proximal end side.
FIG. 10 (b) is a sectional view of the ligation device according to the tenth embodiment attached to the endoscope, illustrating a state that the sliding portion is located on a distal end side.
FIG. 11 (a) is a sectional view of a ligation device according to the eleventh embodiment attached to an endoscope, illustrating a state that a sliding portion is located on a proximal end side.
FIG. 11 (b) is a sectional view of the ligation device according to the eleventh embodiment attached to the endoscope, illustrating a state that the sliding portion is located on a distal end side.
FIG. 12 (a) is a sectional view of a ligation device according to the twelfth embodiment attached to an endoscope, illustrating a state that a sliding portion is located on a proximal end side.
FIG. 12 (b) is a sectional view of the ligation device according to the twelfth embodiment attached to the endoscope, illustrating a state that the sliding portion is located on a distal end side.
FIG. 13 is a sectional view of a ligation device according to the thirteenth embodiment attached to an endoscope, illustrating a state that a sliding portion is located on a proximal end side.
FIG. 14 is a sectional view of a ligation device according to the fourteenth embodiment attached to an endoscope, illustrating a state that a sliding portion is located on a proximal end side.
FIG. 15 is a partially cut-out sectional view of a ligation device according to the fifteenth embodiment including an endoscope portion.

### DESCRIPTION OF EMBODIMENTS

Hereinafter, the embodiments of the present invention will be explained with reference to the figures. Note that sizes of the ligation device illustrated in the figures are described to make it easier to understand the contents of the embodiments, and do not correspond to the actual sizes.

### <First Embodiment>

The first embodiment of the present invention will be explained with reference to the figures.

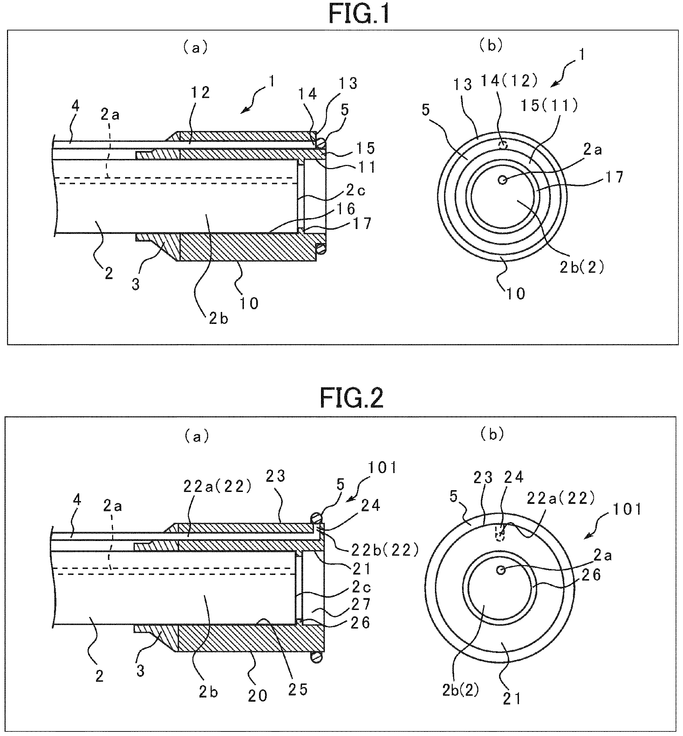

FIG. 1 (a) is a sectional view of a ligation device 1 according to the first embodiment of the present invention attached to an endoscope 2. FIG. 1(b) is a front view of the ligation device 1 attached to the endoscope 2. FIG. 1(a) illustrates only a distal end portion of the endoscope 2 equipped with the ligation device 1.

Additionally, in FIG. 1 (a), the right side of the figure is a distal end side (farther side) where the device is to be inserted into a body, and the left side is a proximal end side (hand side, nearer side). The endoscope 2 has a forceps hole 2a through which forceps not illustrated are inserted. Note that, for the endoscope 2, its appearance is illustrated, and its sectional view is not illustrated (the same applies to the following figures).

The ligation device 1 includes an attachment cylinder 3, a main body 10, a tube 4, and a first ligation ring 5.

The attachment cylinder 3 is cylindrical and placed for fixing the ligation device 1 to the endoscope 2. A material constituting the attachment cylinder 3 is not particularly limited as long as the material has an appropriate softness allowing the attachment cylinder 3 to be attached to or detached from the endoscope 2 and reliably fixed to the endoscope 2, and is biocompatible. For example, a natural rubber, a synthetic rubber, or an elastic material such as a thermoplastic elastomer can be used. Examples of the synthetic rubber include an isoprene rubber, a butadiene rubber, a styrene-butadiene rubber, a nitrile rubber, a butyl rubber, an ethylene-propylene rubber, an acrylic rubber, a fluorine rubber, a silicone rubber, and the like. In addition, examples of the thermoplastic elastomer include a styrene-based elastomer, an olefin-based elastomer, a polyester-based elastomer, a polyurethane-based elastomer, a polyamide-based elastomer, and the like.

The main body 10 is cylindrical, connected to a distal end of the attachment cylinder 3, located on an endoscope distal end portion 2b, and attached to the endoscope distal end portion 2b in an airtight or liquidtight state. The main body 10 has a distal end portion 11 projecting toward a distal end side of a distal end face 2c of the endoscope 2. The main body 10 has a first fluid feeding passage 12 extending along the axial direction of the main body 10. The first fluid feeding passage 12 has a first opening 14 that opens on a distal end face 13 of the main body 10. The distal end portion 11 of the main body 10 is located on an inner peripheral side of the first opening 14 and has an annular protruding portion 15 projecting toward the distal end side. A projection 17 projecting inward is placed on an inner peripheral face 16 of the main body 10. In the axial direction, a distal end of the endoscope distal end portion 2b abuts on the projection 17. The distal end portion 11 and the distal end face 2c of the endoscope 2 constitute a recessed space 18.

In view of the first ligation ring 5 being attached to the main body 10 as described later, a material constituting the main body 10 is not particularly limited as long as the material has a strength endurable against attaching the first ligation ring 5 and is biocompatible. For example, a metal material, a resin material, and a ceramic material can be used. Typical examples of the metal material include a stainless steel, titanium, and a nickel-titanium alloy. Examples of the resin material include a polyethylene, a polypropylene, a polyvinyl chloride, a polystyrene, an acrylic resin, a phenolic resin, a melamine resin, a polyimide, a polyamide, a polycarbonate, a polyether sulfone, a polyetheretherketone, and a polytetrafluoroethylene. Examples of the ceramic material include glass and fine ceramics. Also, the material constituting the main body 10 may be a translucent material, e.g. a polypropylene, a polycarbonate, a polyethersulfone, a polyimide, or an acrylic resin for maintaining a wide visual field during treatment.

The tube 4 extends from the distal end portion to the proximal end portion along the endoscope 2. A syringe not illustrated, for delivering a fluid such as air to the first fluid feeding passage 12 of the main body 10 is connected to a proximal end of the tube 4. A distal end of the tube 4 is airtightly connected to a proximal end of the first fluid feeding passage 12. A material constituting the tube 4 is not particularly limited as long as the material has softness capable of following deformation of the endoscope 2 and is biocompatible, and examples of the material include a polyethylene, a polypropylene, a polyvinyl chloride, and a fluororesin.

The first ligation ring 5 is an O-ring, which is attached to an outer periphery of the protruding portion 15 of the distal end portion 11 of the main body 10 so as to airtightly obstruct the first opening 14. A material constituting the first ligation ring 5 is not particularly limited as long as the material sufficiently extends so as to be attachable to the distal end portion 11 and has a ligation force sufficient to necrotize a diverticula and is biocompatible. For example, an elastic material such as a natural rubber, a synthetic rubber, or a thermoplastic elastomer and the like can be used. Examples of the synthetic rubber include an isoprene rubber, a butadiene rubber, a styrene-butadiene rubber, a nitrile rubber, a butyl rubber, an ethylene-propylene rubber, an acrylic rubber, a fluorine rubber, a silicone rubber, and the like. In addition, examples of the thermoplastic elastomer include a styrene-based elastomer, an olefin-based elastomer, a polyester-based elastomer, a polyurethane-based elastomer, a polyamide-based elastomer, and the like. Incidentally, the material constituting the first ligation ring 5 may include e.g. gold, platinum, tungsten, an alloy containing these elements (e.g. a platinum-nickel alloy, or the like), a radiopaque material such as barium sulfate, bismuth subcarbonate, bismuth trioxide, bismuth oxychloride, and bismuth subcarbonate, or a powder of the radiopaque material. A sectional shape of the first ligation ring 5 is not limited to circle, and may be another shape such as rectangle. A color of the first ligation ring 5 is preferably a color such as black, which is distinct from a surrounding tissue.

A whole length of the ligation device 1 (length from a distal end of the main body 10 to a rear end of the attachment cylinder 3) is set to e.g. 20 to 25 mm. In the main body 10 (part without the projection 17), an inner diameter is set to e.g. 8 to 16 mm, and an outer diameter (maximum diameter) is set to e.g. 10 to 20 mm.

Next, an example of usage of the ligation device 1 will be explained. Herein, a method of ligating a diverticula formed on a wall face of a digestive tract will be explained as an example.

First, the endoscope 2 equipped with the ligation device 1 is inserted into the digestive tract, and an inside of the digestive tract is observed. A bleeding diverticula is identified, the endoscope 2 and the ligation device 1 are made close to the diverticula, the diverticula is sucked through the forceps hole 2a, the diverticula is reversed so as to protrude toward the endoscope 2, and the diverticula is located in the recessed space 18. A fluid is delivered to the first fluid feeding passage 12 of the main body 10 through the tube 4 by operating the syringe not illustrated, and the first ligation ring 5 is ejected from the main body 10 such that the first ligation ring 5 is pushed out using a pressure of the fluid as a driving source, and the sucked diverticula is ligated with the first ligation ring 5. Then, the endoscope 2 equipped with the ligation device 1 is taken out of the digestive tract. After that, the ligated diverticula necrotizes and is discharged to the outside of the body together with the first ligation ring 5.

In the ligation device 1 according to the first embodiment, the first ligation ring 5 is attached to the distal end portion 11 of the main body 10 so as to airtightly obstruct the first opening 14. Thereby, the first ligation ring 5 can be reliably ejected from the main body 10 such that the first ligation ring 5 is pushed out using the pressure of the fluid flowing through the first fluid feeding passage 12 as a driving source.

The inner peripheral face 16 of the main body 10 has the projection 17 on which the distal end of the endoscope distal end portion 2b abuts. Thus, the projection 17 makes it easier to position the ligation device 1 with respect to the endoscope distal end portion 2b.

The main body 10 is made of a translucent material, so that a wide visual field of the endoscope 2 during treatment can be maintained.

### <Second Embodiment>

The second embodiment of the present invention will be explained with reference to the figures.

FIG. 2 (a) is a sectional view of a ligation device 101 according to the second embodiment of the present invention attached to an endoscope 2. FIG. 2 (b) is a front view of the ligation device 101 attached to the endoscope 2. FIG. 2 (a) illustrates only a distal end portion of the endoscope 2 equipped with the ligation device 101.

Additionally, in FIG. 2 (a), the right side of the figure is a distal end side (farther side) where the device is to be inserted into a body, and the left side is a proximal end side (hand side, nearer side).

Note that, in the ligation device 101 according to the second embodiment, the same members as in the ligation device 1 according to the first embodiment are given the same reference numerals as in the first embodiment, and detailed explanation of the same members is omitted.

The ligation device 101 includes the attachment cylinder 3, a main body 20, the tube 4, and the first ligation ring 5.

The main body 20 is cylindrical, connected to the distal end of the attachment cylinder 3, and located on the endoscope distal end portion 2b. The main body 20 has a distal end portion 21 projecting toward the distal end side of the distal end face 2c of the endoscope 2. The main body 20 has a first fluid feeding passage 22. The first fluid feeding passage 22 has an axial flow passage 22a extending from a proximal end of the main body 20 to the distal end portion 21 along an axial direction, and a radial flow passage 22b extending from a distal end of the axial flow passage 22a to an outer peripheral face 23 along a radial direction. Thus, the first fluid feeding passage 22 has a first opening 24 that opens on the outer peripheral face 23 of the main body 20. A projection 26 projecting inward is placed on an inner peripheral face 25 of the main body 20. In the axial direction, the distal end of the endoscope distal end portion 2b abuts on the projection 26. The distal end portion 21 and the distal end face 2c of the endoscope 2 constitute a recessed space 27. Incidentally, since a material constituting the main body 20 is the same as the material constituting the main body 10 according to the first embodiment, the explanation in the first embodiment is applied to explanation of the material for the main body 20, and detailed explanation of the material for the main body 20 is omitted.

The first ligation ring 5 is attached to the outer peripheral face 23 of the main body 20 so as to airtightly obstruct the first opening 24.

In the ligation device 101 according to the second embodiment, the first ligation ring 5 is attached to the distal end portion 21 of the main body 20 so as to airtightly obstruct the first opening 24. Thereby, the first ligation ring 5 can be reliably ejected from the main body 20 using a pressure of a fluid flowing through the first fluid feeding passage 22 as a driving source.

### <Third Embodiment>

The third embodiment of the present invention will be explained with reference to the figures.

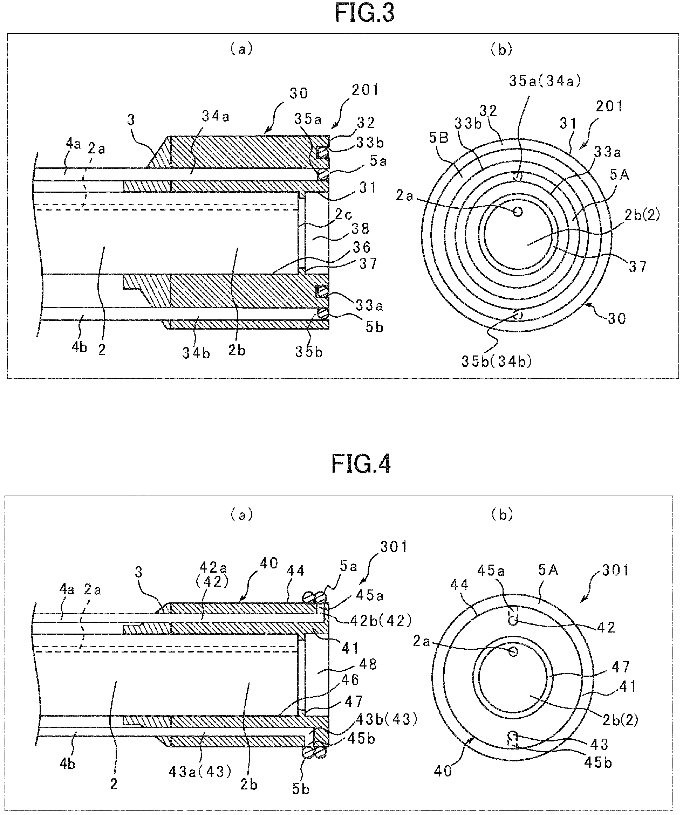

FIG. 3 (a) is a sectional view of a ligation device 201 according to the third embodiment of the present invention attached to the endoscope 2. FIG. 3 (b) is a front view of the ligation device 201 attached to the endoscope 2. FIG. 3 (a) illustrates only the distal end portion of the endoscope 2 equipped with the ligation device 201.

Additionally, in FIG. 3 (a), the right side of the figure is a distal end side (farther side) where the device is to be inserted into a body, and the left side is a proximal end side (hand side, nearer side).

Note that, in the ligation device 201 according to the third embodiment, the same members as in the ligation device 1 according to the first embodiment are given the same reference numerals as in the first embodiment, and detailed explanation of the same members is omitted.

The ligation device 201 includes the attachment cylinder 3, a main body 30, a first tube 4a, a second tube 4b, a first ligation ring 5a, and a second ligation ring 5b.

The main body 30 is cylindrical, connected to the distal end of the attachment cylinder 3, and located on the endoscope distal end portion 2b. The main body 30 has a distal end portion 31 projecting toward the distal end side of the distal end face 2c of the endoscope 2. A distal end face 32 of the distal end portion 31 has a first annular recessed portion 33a and a second annular recessed portion 33b. The first recessed portion 33a and the second recessed portion 33b are coaxial with the main body 30 and have different diameters. The first recessed portion 33a is configured to have a diameter smaller than of the second recessed portion 33b.

The main body 30 has a first fluid feeding passage 34a and a second fluid feeding passage 34b that extend along the axial direction. The first fluid feeding passage 34a and the second fluid feeding passage 34b are formed at positions opposite to each other with respect to the axis. The first fluid feeding passage 34a has a first opening 35a that opens on the distal end face 32 through the first recessed portion 33a. The second fluid feeding passage 34b has a second opening 35b that opens on the distal end face 32 through the second recessed portion 33b. The first tube 4a is connected to a proximal end of the first fluid feeding passage 34a, and the second tube 4b is connected to a proximal end of the second fluid feeding passage 34b.

An inner peripheral face 36 of the main body 30 has a projection 37 projecting inward. In the axial direction, the distal end of the endoscope distal end portion 2b abuts on the projection 37. The distal end portion 31 and the distal end face 2c of the endoscope 2 constitute a recessed space 38. Incidentally, since a material constituting the main body 30 is the same as the material constituting the main body 10 according to the first embodiment, the explanation in the first embodiment is applied to explanation of the material for the main body 30, and detailed explanation of the material for the main body 30 is omitted.

The first ligation ring 5a is attached to the first recessed portion 33a so as to airtightly obstruct the first opening 35a of the first fluid feeding passage 34a. The second ligation ring 5b is attached to the second recessed portion 33b so as to airtightly obstruct the second opening 35b of the second fluid feeding passage 34b.

In the ligation device 201 according to the third embodiment, the first ligation ring 5a is attached to the distal end portion 31 of the main body 30 so as to airtightly obstruct the first opening 35a, and the second ligation ring 5b is attached to the distal end portion 31 of the main body 30 so as to airtightly obstruct the second opening 35b. Thereby, after one affected part is ligated with the first ligation ring 5a, another affected part can be ligated with the second ligation ring 5b. Thus, the ligation treatment can be continuously performed without taking out the ligation device 201 from the body.

### <Fourth Embodiment>

The fourth embodiment of the present invention will be explained with reference to the figures.

FIG. 4 (a) is a sectional view of a ligation device 301 according to the fourth embodiment of the present invention attached to the endoscope 2. FIG. 4 (b) is a front view of the ligation device 301 attached to the endoscope 2. FIG. 4 (a) illustrates only the distal end portion of the endoscope 2 equipped with the ligation device 301.

Additionally, in FIG. 4 (a), the right side of the figure is a distal end side (farther side) where the device is to be inserted into a body, and the left side is a proximal end side (hand side, nearer side).

Note that, in the ligation device 301 according to the fourth embodiment, the same members as in the ligation device 1 according to the first embodiment are given the same reference numerals as in the first embodiment, and detailed explanation of the same members is omitted.

The ligation device 301 includes the attachment cylinder 3, a main body 40, the first tube 4a, the second tube 4b, the first ligation ring 5a, and the second ligation ring 5b.

The main body 40 is cylindrical, connected to the distal end of the attachment cylinder 3, and located on the endoscope distal end portion 2b. The main body 40 has a distal end portion 41 projecting toward the distal end side of the distal end face 2c of the endoscope 2. The main body 40 has a first fluid feeding passage 42 and a second fluid feeding passage 43. The first fluid feeding passage 42 and the second fluid feeding passage 43 are formed at positions opposite to each other with respect to the axis.

The first fluid feeding passage 42 has a first axial flow passage 42a extending from a proximal end of the main body 40 to the distal end portion 41 along the axial direction, and a first radial flow passage 42b extending from a distal end of the first axial flow passage 42a to an outer peripheral face 44 along the radial direction. Thus, the first fluid feeding passage 42 has a first opening 45a that opens on the outer peripheral face 44 of the distal end portion 41 of the main body 40. The first tube 4a is connected to a proximal end of the first fluid feeding passage 42.

The second fluid feeding passage 43 has a second axial flow passage 43a extending from the proximal end of the main body 40 to the distal end portion 41 along the axial direction, and a second radial flow passage 43b extending from a distal end of the second axial flow passage 43a to an outer peripheral face 44 along the radial direction. Thus, the second fluid feeding passage 43 has a second opening 45b that opens on the outer peripheral face 44 of the distal end portion 41 of the main body 40. The second radial flow passage 43b is located on the proximal end side of the first radial flow passage 42b in the axial direction. The second tube 4b is connected to a proximal end of the second fluid feeding passage 43.

An inner peripheral face 46 of the main body 40 has a projection 47 projecting inward. In the axial direction, the distal end of the endoscope distal end portion 2b abuts on the projection 47. The distal end portion 41 and the distal end face 2c of the endoscope 2 constitute a recessed space 48. Incidentally, since a material constituting the main body 40 is the same as the material constituting the main body 10 according to the first embodiment, the explanation in the first embodiment is applied to explanation of the material for the main body 40, and detailed explanation of the material for the main body 40 is omitted.

The first ligation ring 5a is placed so as to airtightly obstruct the first opening 45a of the first fluid feeding passage 42. The second ligation ring 5b is placed so as to airtightly obstruct the second opening 45b of the second fluid feeding passage 43. The second ligation ring 5b is located on the proximal end side of the first ligation ring 5a.

In the ligation device 301 according to the fourth embodiment, the first ligation ring 5a is attached to the distal end portion 41 of the main body 40 so as to airtightly obstruct the first opening 45a, and the second ligation ring 5b is attached to the distal end portion 41 of the main body 40 so as to airtightly obstruct the second opening 45b. Thereby, after one affected part is ligated with the first ligation ring 5a, another affected part can be ligated with the second ligation ring 5b. Thus, the ligation treatment can be continuously performed without taking out the ligation device 301 from the body.

### <Fifth Embodiment>

The fifth embodiment of the present invention will be explained with reference to the figures.

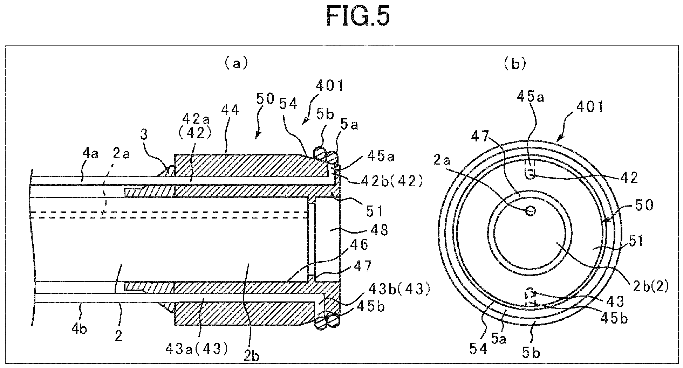

FIG. 5 (a) is a sectional view of a ligation device 401 according to the fifth embodiment of the present invention attached to the endoscope 2. FIG. 5 (b) is a front view of the ligation device 401 attached to the endoscope 2. FIG. 5 (a) illustrates only the distal end portion of the endoscope 2 equipped with the ligation device 401.

Additionally, in FIG. 5 (a), the right side of the figure is a distal end side (farther side) where the device is to be inserted into a body, and the left side is a proximal end side (hand side, nearer side).

Note that, in the ligation device 401 according to the fifth embodiment, the same members as in the ligation device 301 according to the fourth embodiment are given the same reference numerals as in the fourth embodiment, and detailed explanation of the same members is omitted.

The ligation device 401 includes the attachment cylinder 3, a main body 50, the first tube 4a, the second tube 4b, the first ligation ring 5a, and the second ligation ring 5b.

Since the main body 50 has the almost same configuration as of the main body 40 according to the fourth embodiment, the same components as in the fourth embodiment are given the same reference numerals as in the fourth embodiment, and only different components will be explained. A distal end portion 51 of the main body 50 has a tapered shape that tapers toward the distal end. The first opening 45a of the first fluid feeding passage 42 opens on an outer peripheral face 54 of the distal end portion 51 of the main body 50, and the second opening 45b of the second fluid feeding passage 43 opens on the outer peripheral face 54 of the distal end portion 51 of the main body 50.

The first ligation ring 5a is placed so as to airtightly obstruct the first opening 45a of the first fluid feeding passage 42. The second ligation ring 5b is placed so as to airtightly obstruct the second opening 45b of the second fluid feeding passage 43.

In the ligation device 401 according to the fifth embodiment, the first opening 45a and the second opening 45b open on the outer peripheral face 54 of the tapered distal end portion 51, the first ligation ring 5a and the second ligation ring 5b are attached to the outer peripheral face 54, and therefore the first ligation ring 5a and the second ligation ring 5b can be more reliably ejected from the main body.

### <Sixth Embodiment>

The sixth embodiment of the present invention will be explained with reference to the figures.

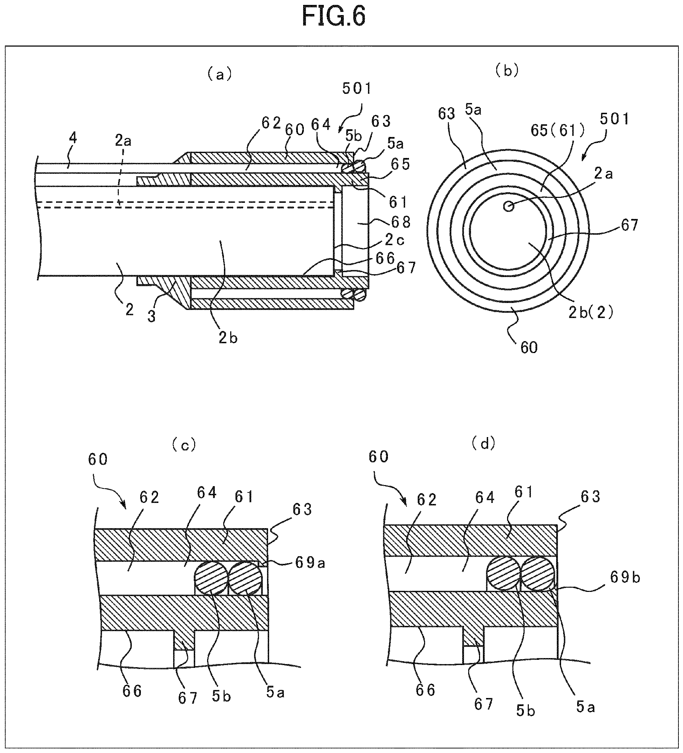

FIG. 6 (a) is a sectional view of a ligation device 501 according to the sixth embodiment of the present invention attached to the endoscope 2. FIG. 6 (b) is a front view of the ligation device 501 attached to the endoscope 2. FIG. 6 (a) illustrates only the distal end portion of the endoscope 2 equipped with the ligation device 501.

Additionally, in FIG. 6 (a), the right side of the figure is a distal end side (farther side) where the device is to be inserted into a body, and the left side is a proximal end side (hand side, nearer side).

Note that, in the ligation device 501 according to the sixth embodiment, the same members as in the ligation device 1 according to the first embodiment are given the same reference numerals as in the first embodiment, and detailed explanation of the same members is omitted.

The ligation device 501 includes the attachment cylinder 3, a main body 60, the tube 4, the first ligation ring 5a, and the second ligation ring 5b.

The main body 60 is cylindrical, connected to the distal end of the attachment cylinder 3, and located on the endoscope distal end portion 2b. The main body 60 has a distal end portion 61 projecting toward the distal end side of the distal end face 2c of the endoscope 2. The main body 60 has a first fluid feeding passage 62 extending along the axial direction of the main body 60. The first fluid feeding passage 62 is formed so as to have a cylindrical shape coaxial with the main body 60, and has a first annular opening 64 that opens on a distal end face 63 of the main body 60 and is coaxial with the main body 60. The distal end portion 61 of the main body 60 is located on an inner peripheral side of the first opening 64 and has an annular protruding portion 65 projecting toward the distal end side. An inner peripheral face 66 of the main body 60 has a projection 67 projecting inward. In the axial direction, the distal end of the endoscope distal end portion 2b abuts on the projection 67. The distal end portion 61 and the distal end face 2c of the endoscope 2 constitute a recessed space 68.

The first ligation ring 5a is attached to the outer periphery of the protruding portion 15 on the distal end portion 11 of the main body 60 so as to airtightly obstruct the first annular opening 64. The second ligation ring 5b is attached to the proximal end side of the first ligation ring 5a on the first opening 64.

In the ligation device 501 according to the sixth embodiment, a fluid is delivered to the first fluid feeding passage 62 of the main body 60 through the tube 4, and the second ligation ring 5b is pushed using a pressure of the fluid as a driving source. When the second ligation ring 5b is pushed, the first ligation ring 5a is pushed out, so that the first ligation ring 5a can be ejected from the main body 60. Subsequently, the second ligation ring 5b is pushed using the pressure of the fluid as a driving source, so that the second ligation ring 5b can be ejected from the main body 60. In this way, the ligation treatment can be continuously performed without taking out the ligation device 501 from the body.

In addition, as illustrated in FIG. 6 (c) and FIG. 6 (d), the distal end portion 61 of the main body 60 may be configured to cover the outer periphery of the first ligation ring 5a. As illustrated in FIG. 6 (c), a stopper 69a projecting inward on the distal end of the outer peripheral portion of the part constituting the first fluid feeding passage 62 may be placed on the distal end portion 61 of the main body 60. Alternatively, as illustrated in FIG. 6 (c), a stopper 69b projecting outward on the distal end of the inner peripheral portion of the part constituting the first fluid feeding passage 62 may be placed on the distal end portion 61 of the main body 60. This configuration makes it possible to reliably eject the first ligation ring 5a and the second ligation ring 5b one by one from the main body 60.

### <Seventh Embodiment>

The seventh embodiment of the present invention will be explained with reference to the figures.

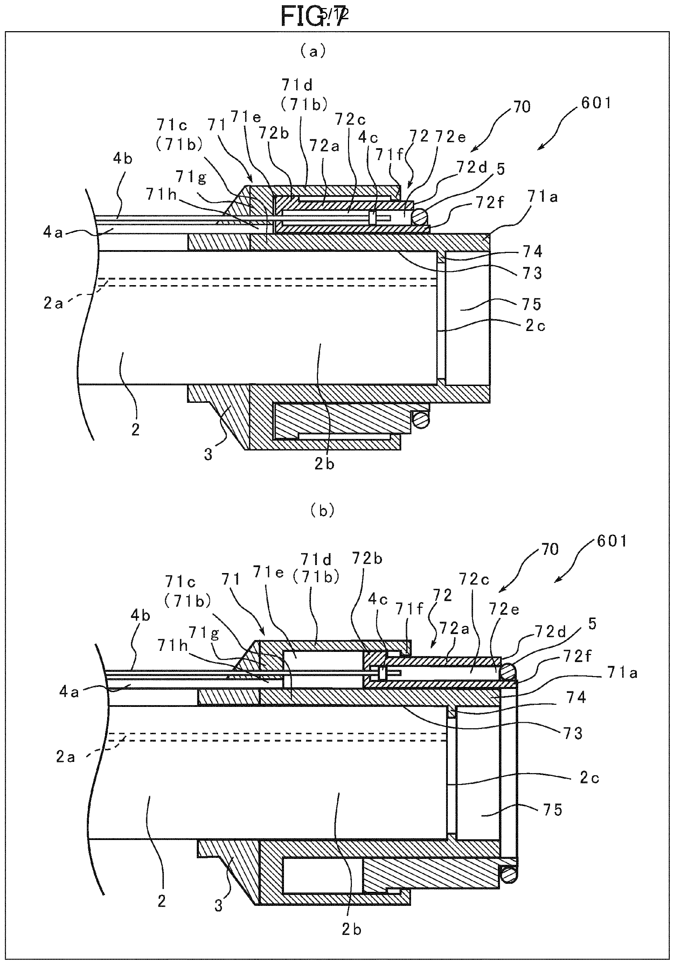

FIG. 7 (a) is a sectional view of a ligation device 601 according to the seventh embodiment of the present invention attached to the endoscope 2, illustrating a state that a sliding portion 72 is located on the proximal end side. FIG. 7 (b) is a sectional view of the ligation device 601 attached to the endoscope 2, illustrating a state that the sliding portion 72 is located on the distal end side. FIG. 7 (a) and FIG. 7 (b) illustrate only the distal end portion of the endoscope 2 equipped with the ligation device 601.

Additionally, in FIG. 7 (a) and FIG. 7 (b), the right side of the figure is a distal end side (farther side) where the device is to be inserted into a body, and the left side is a proximal end side (hand side, nearer side).

Note that, in the ligation device 601 according to the seventh embodiment, the same members as in the ligation device 1 according to the first embodiment are given the same reference numerals as in the first embodiment, and detailed explanation of the same members is omitted.

The ligation device 601 includes the attachment cylinder 3, a main body 70, the first tube 4a, the second tube 4b, and the first ligation ring 5.

The main body 70 has a cylindrical base portion 71 and a cylindrical sliding portion 72, and is connected to the distal end of the attachment cylinder 3, and located on the endoscope distal end portion 2b.

The base portion 71 has a distal end portion 71a projecting toward the distal end side of the distal end face 2c of the endoscope 2, and a proximal end portion 71b. The proximal end portion 71b has an inner peripheral portion 71c and an outer peripheral portion 71d. The inner peripheral portion 71c and the outer peripheral portion 71d constitute an annular slide space 71e. An annular protruding portion 71f projecting inward is placed on a distal end of the outer peripheral portion 71d. The slide space 71e opens in an annular shape on the distal end side by the protruding portion 71f and the inner peripheral portion 71c. A proximal end portion 71g of the outer peripheral portion 71d projects inward and is connected to the inner peripheral portion 71c. The proximal end portion 71g has a through-hole 71h.

The sliding portion 72 has a cylindrical portion 72a and a flange portion 72b. The cylindrical portion 72a has a first fluid feeding passage 72c along the axial direction. The first fluid feeding passage 72c has a first opening 72e that opens on a distal end face 72d of the sliding portion 72. The cylindrical portion 72a of the sliding portion 72 is located on the inner peripheral side of the first opening 72e and has an annular protruding portion 72f projecting toward the distal end side. The cylindrical portion 72a is configured to have an axial-direction length larger than an axial-direction length of the slide space 71e, and the protruding portion 72f of the cylindrical portion 72a is always located on the distal end side of the slide space 71e.

The flange portion 72b is placed so as to project outward in the radial direction on the proximal end portion of the cylindrical portion 72a. In a state that the proximal end side of the cylindrical portion 72a and the flange portion 72b are inserted into the slide space 71e, airtightness of an annular space formed by the outer peripheral portion 71d, the inner peripheral portion 71c, and a rear end of the sliding portion 72 is structurally maintained. The flange portion 72b of the sliding portion 72 is located in the slide space 71e, and slidably attached to the base portion 71. As illustrated in FIG. 7 (a) and 7 (b), the sliding portion 72 is configured such that, when the sliding portion 72 slides with respect to the base portion 71, the protruding portion 72f of the sliding portion 72 moves to the proximal end side of the distal end of the distal end portion 71a and the distal end side of the distal end of the distal end portion 71a. In a state that the sliding portion 72 is located on the most distal end side, the flange portion 72b abuts on the protruding portion 71f, and the sliding portion 72 is prevented from coming out of the base portion 71.

An inner peripheral face 73 of the main body 70 has a projection 74 projecting inward. The distal end of the endoscope distal end portion 2b abuts on the projection 74 in the axial direction. The distal end portion 71a and the distal end face 2c of the endoscope 2 constitute a recessed space 75. Amaterial constituting the main body 70 is not particularly limited as long as the material has excellent slidability between the base portion 71 and the sliding portion 72, and has a mechanical strength endurable against an elastic force of the first ligation ring 5 when the sliding portion 72 slides to the distal end side, and is biocompatible. Examples of the material include the materials cited as the materials for the main body 10.

The first tube 4a is connected to the proximal end of the through-hole 71g. The second tube 4b penetrates the proximal end portion of the cylindrical portion 72a of the sliding portion 72 to extend into the first fluid feeding passage 72c. A sealing member 4c is attached to the distal end of the second tube 4b. The second tube 4b is fixed to the attachment cylinder 3 and the base portion 71, and the sliding portion 72 can slidably move with respect to the sealing member 4c. In the first fluid feeding passage 72c, the sealing member 4c maintains airtightness between the spaces on the front and back of the sealing member 4c. The sealing member 4c is attached to the second tube 4b at a position where sliding of the sliding portion 72 is not inhibited.

The first ligation ring 5 is attached to the protruding portion 72f of the sliding portion 72 so as to airtightly obstruct the first opening 72e.

A whole length of the ligation device 701 (length from the distal end of the inner cylinder 20 to the rear end of the joining member 10) is set to e.g. 20 to 25 mm. In an inner cylinder 20b (part without the projection 26), e.g. an inner diameter is set to 8 to 16 mm, and an outer diameter (maximum diameter) is set to 11 to 25 mm.

In the ligation device 601 according to the seventh embodiment, when searching for an affected part such as diverticula, the sliding portion 72 is sucked by the first tube 4a, so that the sliding portion 72 is located on the proximal end side, as illustrated in FIG. 7 (a). During ligation, the fluid is fed to the slide space 71e through the first tube 4a, so that the sliding portion 72 is moved to the distal end side, as illustrated in FIG. 7 (b). In this state, the fluid is delivered to the first fluid feeding passage 72c of the sliding portion 72 of the main body 70 through the second tube 4b, the first ligation ring 5 is ejected from the main body 70 such that the first ligation ring 5 is pushed out using a pressure of the fluid as a driving source, and the affected part is ligated with the first ligation ring 5. In this way, when searching for the affected part, the sliding portion 72 is located on the proximal end side, so that a wide visual field of the endoscope 2 can be maintained, and, during ligation, the sliding portion 72 is moved to the distal end side to allow the ligation.

### <Eighth Embodiment>

The eighth embodiment of the present invention will be explained with reference to the figures.

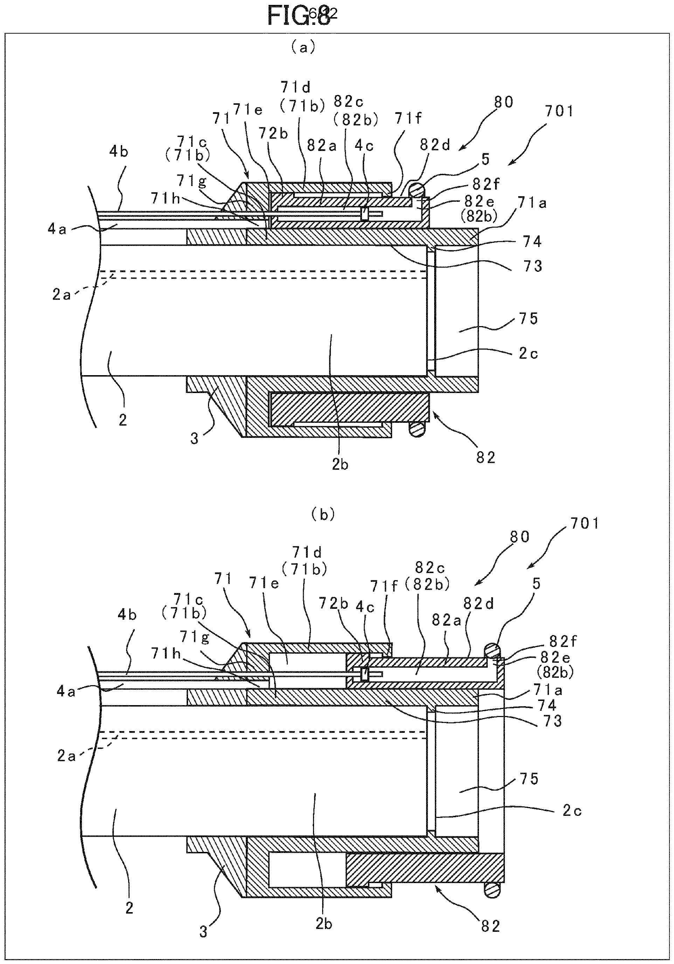

FIG. 8 (a) is a sectional view of a ligation device 701 according to the eighth embodiment of the present invention attached to the endoscope 2, illustrating a state that a sliding portion 82 is located on the proximal end side. FIG. 8 (b) is a sectional view of the ligation device 701 attached to the endoscope 2, illustrating a state that the sliding portion 82 is located on the distal end side. FIG. 8 (a) and FIG. 8 (b) illustrate only the distal end portion of the endoscope 2 equipped with the ligation device 701.

Additionally, in FIG. 8 (a) and FIG. 8 (b), the right side of the figure is a distal end side (farther side) where the device is to be inserted into a body, and the left side is a proximal end side (hand side, nearer side).

Note that, in the ligation device 701 according to the eighth embodiment, the same members as in the ligation device 601 according to the seventh embodiment are given the same reference numerals as in the seventh embodiment, and detailed explanation of the same members is omitted.

The ligation device 701 includes the attachment cylinder 3, a main body 80, the first tube 4a, the second tube 4b, and the first ligation ring 5.

The main body 80 has the cylindrical base portion 71 and the cylindrical sliding portion 82, and is connected to the distal end of the attachment cylinder 3, and located on the endoscope distal end portion 2b.

The sliding portion 82 has a cylindrical portion 82a and the flange portion 72b. The cylindrical portion 82a has a first fluid feeding passage 82b. The first fluid feeding passage 82b has an axial flow passage 82c extending from a proximal end of the cylindrical portion 82a along the axial direction, and a radial flow passage 82e extending from a distal end of the axial flow passage 82c to an outer peripheral face 82d along the radial direction. Thus, the first fluid feeding passage 82b has a first opening 82f that opens on the outer peripheral face 82d of the sliding portion 82. The cylindrical portion 82a is configured to have an axial-direction length larger than the axial-direction length of the slide space 71e, and the first opening 82f of the cylindrical portion 82a is always located on the distal end side of the slide space 71e.

In a state that the proximal end side of the cylindrical portion 82a and the flange portion 72b are inserted into the slide space 71e, airtightness of an annular space formed by the outer peripheral portion 71d, the inner peripheral portion 71c, and a rear end of the sliding portion 82 is structurally maintained. In the sliding portion 82, the flange portion 72b is located in the slide space 71e, and the sliding portion 82 is slidably attached to the base portion 71. As illustrated in FIG. 8 (a) and 8 (b), the sliding portion 82 is configured such that, when the sliding portion 82 slides with respect to the base portion 71, the first opening 82f of the sliding portion 82 moves to the proximal end side of the distal end of the distal end portion 71a and the distal end side of the distal end of the distal end portion 71a. Incidentally, since a material constituting the main body 80 is the same as the material constituting the main body 70 according to the seventh embodiment, the explanation in the seventh embodiment is applied to explanation of the material for the main body 80, and detailed explanation of the material for the main body 80 is omitted.

The second tube 4b penetrates the proximal end of the cylindrical portion 82a of the sliding portion 82 to extend into the first fluid feeding passage 82b.

The first ligation ring 5 is attached to the outer peripheral face 82d of the sliding portion 82 of the main body 80 so as to airtightly obstruct the first opening 82f.

In the ligation device 701 according to the eighth embodiment, when searching for an affected part such as diverticula, the sliding portion 82 is sucked by the first tube 4a, so that the sliding portion 82 is located on the proximal end side, as illustrated in FIG. 8 (a). During ligation, the fluid is fed to the slide space 71e through the first tube 4a, so that the sliding portion 82 is moved to the distal end side, as illustrated in FIG. 8 (b). In this state, the fluid is delivered to the first fluid feeding passage 82b of the sliding portion 82 of the main body 80 through the second tube 4b, the first ligation ring 5 is ejected from the main body 80 using a pressure of the fluid as a driving source, and the affected part is ligated with the first ligation ring 5. In this way, when searching for the affected part, the sliding portion 82 is located on the proximal end side, so that a wide visual field of the endoscope 2 can be maintained, and, during ligation, the sliding portion 82 is moved to the distal end side to allow the ligation.

### <Ninth Embodiment>

The ninth embodiment of the present invention will be explained with reference to the figures.

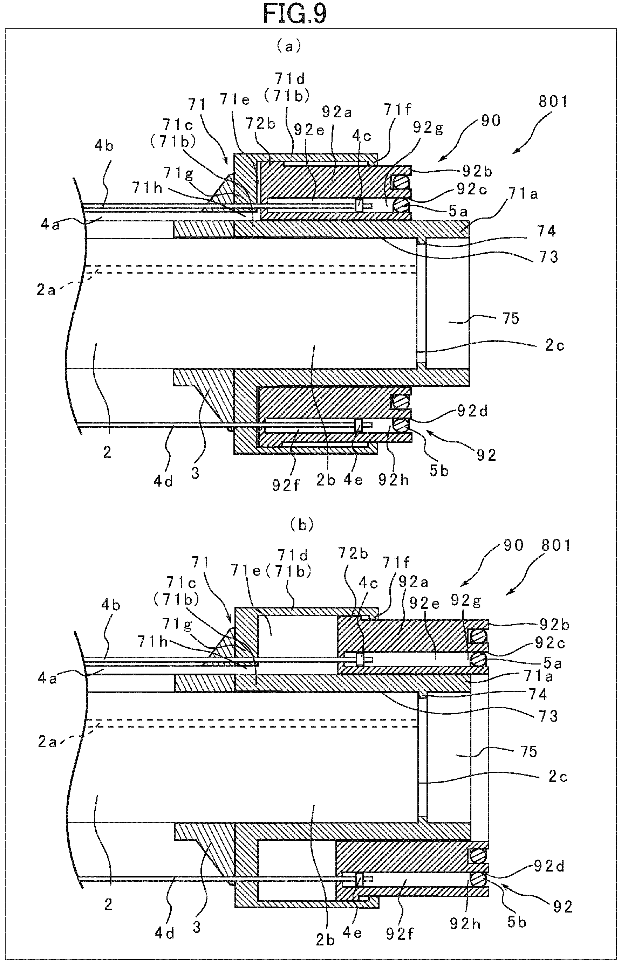

FIG. 9 (a) is a sectional view of a ligation device 801 according to the ninth embodiment of the present invention attached to the endoscope 2, illustrating a state that a sliding portion 92 is located on the proximal end side. FIG. 9 (b) is a sectional view of the ligation device 801 attached to the endoscope 2, illustrating a state that the sliding portion 92 is located on the distal end side. FIG. 9 (a) and FIG. 9 (b) illustrate only the distal end portion of the endoscope 2 equipped with the ligation device 801.

Additionally, in FIG. 9 (a) and FIG. 9 (b), the right side of the figure is a distal end side (farther side) where the device is to be inserted into a body, and the left side is a proximal end side (hand side, nearer side).

Note that, in the ligation device 801 according to the ninth embodiment, the same members as in the ligation device 601 according to the seventh embodiment are given the same reference numerals as in the seventh embodiment, and detailed explanation of the same members is omitted.

The ligation device 801 includes the attachment cylinder 3, a main body 90, the first tube 4a, the second tube 4b, a third tube 4d, the first ligation ring 5a, and the second ligation ring 5b.

The main body 90 has the cylindrical base portion 71 and the cylindrical sliding portion 92, and is connected to the distal end of the attachment cylinder 3, and located on the endoscope distal end portion 2b.

The sliding portion 92 has a cylindrical portion 92a and the flange portion 72b. A distal end face 92b of the cylindrical portion 92a has a first annular recessed portion 92c and a second annular recessed portion 92d. The first recessed portion 92c and the second recessed portion 92d are coaxial with the cylindrical portion 92a and have different diameters. The first recessed portion 92c is configured to have a diameter smaller than of the second recessed portion 92d.

The cylindrical portion 92a has a first fluid feeding passage 92e and a second fluid feeding passage 92f that extend along the axial direction. The first fluid feeding passage 92e and the second fluid feeding passage 92f are formed at positions opposite to each other with respect to the axis. The first fluid feeding passage 92e has a first opening 92g that opens on the distal end face 92b through the first recessed portion 92c. The second fluid feeding passage 92f has a second opening 92h that opens on the distal end face 92b through the second recessed portion 92d. The cylindrical portion 92a is configured to have an axial-direction length larger than the axial-direction length of the slide space 71e. The first opening 92g and the second opening 92h of the cylindrical portion 92a are always located on the distal end side of the slide space 71e.

In a state that the proximal end side of the cylindrical portion 92a and the flange portion 72b are inserted into the slide space 71e, airtightness of an annular space formed by the outer peripheral portion 71d, the inner peripheral portion 71c, and a rear end of the sliding portion 92 is structurally maintained. In the sliding portion 92, the flange portion 72b is located in the slide space 71e, and slidably attached to the base portion 71. As illustrated in FIG. 9 (a) and 9 (b), the sliding portion 92 is configured such that, when the sliding portion 92 slides with respect to the base portion 71, the first opening 92g and the second opening 92h of the sliding portion 92 move to the proximal end side of the distal end of the distal end portion 71a and the distal end side of the distal end of the distal end portion 71a. Incidentally, since a material constituting the main body 90 is the same as the material constituting the main body 70 according to the seventh embodiment, the explanation in the seventh embodiment is applied to explanation of the material for the main body 90, and detailed explanation of the material for the main body 90 is omitted.

The second tube 4b penetrates the proximal end portion of the cylindrical portion 92a of the sliding portion 92 to extend into the first fluid feeding passage 92e. The sealing member 4c is attached to the distal end of the second tube 4b. The second tube 4b is fixed to the attachment cylinder 3 and the base portion 71, and the sliding portion 92 can slidably move with respect to the sealing member 4c. In the first fluid feeding passage 92e, the sealing member 4c maintains airtightness between the spaces on the front and back of the sealing member 4c. The sealing member 4c is attached to the second tube 4b at a position where sliding of the sliding portion 92 is not inhibited.

The third tube 4d penetrates the proximal end portion of the cylindrical portion 92a of the sliding portion 92 to extend into the second fluid feeding passage 92f. A sealing member 4e is attached to the distal end of the third tube 4d. The third tube 4d is fixed to the attachment cylinder 3 and the base portion 71, and the sliding portion 92 can slidably move with respect to the sealing member 4e. In the second fluid feeding passage 92f, the sealing member 4e maintains airtightness between the spaces on the front and back of the sealing member 4e. The sealing member 4e is attached to the second tube 4b at a position where sliding of the sliding portion 92 is not inhibited.

The first ligation ring 5a is attached to the first recessed portion 92c so as to airtightly obstruct the first opening 92g of the first fluid feeding passage 92e. The second ligation ring 5b is attached to the second recessed portion 92d so as to airtightly obstruct the second opening 92h of the second fluid feeding passage 92f.

In the ligation device 801 according to the ninth embodiment, when searching for an affected part such as diverticula, the sliding portion 92 is sucked by the first tube 4a, so that the sliding portion 92 is located on the proximal end side, as illustrated in FIG. 9 (a). During ligation, the fluid is fed to the slide space 71e through the first tube 4a, so that the sliding portion 92 is moved to the distal end side, as illustrated in FIG. 9 (b). In this state, the fluid is delivered to the first fluid feeding passage 92e of the sliding portion 92 of the main body 90 through the second tube 4b, the first ligation ring 5a is ejected from the main body 90 using a pressure of the fluid as a driving source, and the affected part is ligated with the first ligation ring 5a. In this way, when searching for the affected part, the sliding portion 92 is located on the proximal end side, so that a wide visual field of the endoscope 2 can be maintained, and, during ligation, the sliding portion 92 is moved to the distal end side to allow the ligation.

Furthermore, after ligating one affected part with the first ligation ring 5a, the sliding portion 92 is sucked by the first tube 4a, so that the sliding portion 92 is located on the proximal end side, and another affected part is identified. The fluid is delivered to the second fluid feeding passage 92f of the sliding portion 92 of the main body 90 through the third tube 4d, and the second ligation ring 5b is ejected from the main body 90 using a pressure of the fluid as a driving source, and another affected part is ligated with the second ligation ring 5b. Thus, the ligation treatment can be continuously performed without taking out the ligation device 901 from the body.

### <Tenth Embodiment>

The tenth embodiment of the present invention will be explained with reference to the figures.

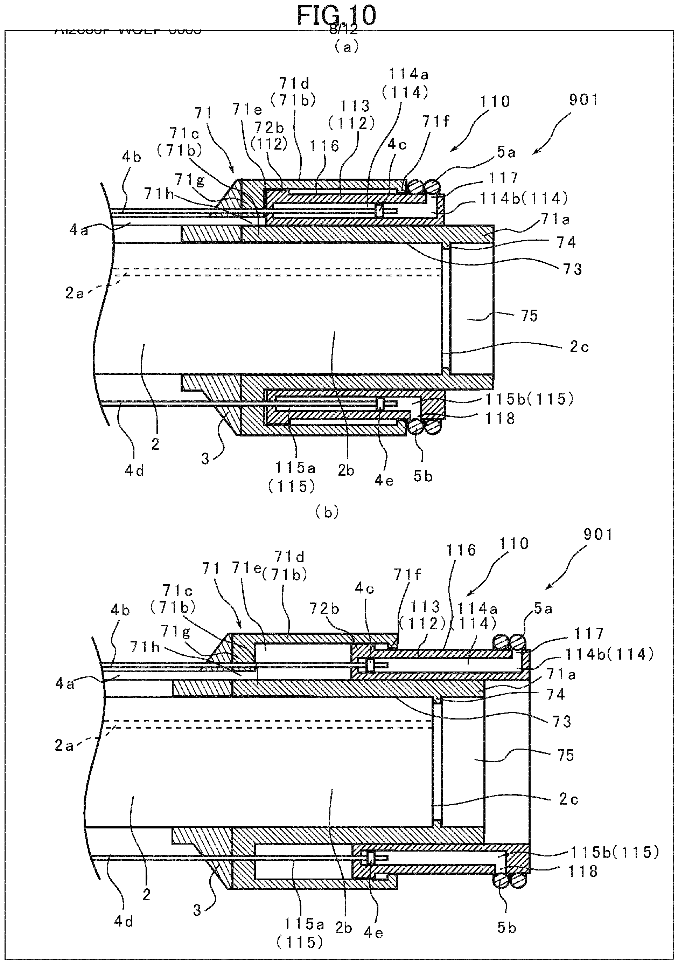

FIG. 10 (a) is a sectional view of a ligation device 901 according to the tenth embodiment of the present invention attached to the endoscope 2, illustrating a state that a sliding portion 112 is located on the proximal end side. FIG. 10 (b) is a sectional view of the ligation device 901 attached to the endoscope 2, illustrating a state that the sliding portion 112 is located on the distal end side. FIG. 10 (a) and FIG. 10 (b) illustrate only the distal end portion of the endoscope 2 equipped with the ligation device 901.

Additionally, in FIG. 10 (a) and FIG. 10 (b), the right side of the figure is a distal end side (farther side) where the device is to be inserted into a body, and the left side is a proximal end side (hand side, nearer side).

Note that, in the ligation device 901 according to the tenth embodiment, the same members as in the ligation device 601 according to the seventh embodiment are given the same reference numerals as in the seventh embodiment, and detailed explanation of the same members is omitted.

The ligation device 901 includes the attachment cylinder 3, a main body 110, the first tube 4a, the second tube 4b, the third tube 4d, the first ligation ring 5a, and the second ligation ring 5b.

The main body 110 has the cylindrical base portion 71 and the cylindrical sliding portion 112, and is connected to the distal end of the attachment cylinder 3, and located on the endoscope distal end portion 2b.

The sliding portion 112 has a cylindrical portion 113 and the flange portion 72b. The cylindrical portion 113 has a first fluid feeding passage 114 and a second fluid feeding passage 115. The first fluid feeding passage 114 and the second fluid feeding passage 115 are formed at positions opposite to each other with respect to the axis.

The first fluid feeding passage 114 has a first axial flow passage 114a extending from a proximal end of the cylindrical portion 113 along the axial direction, and a first radial flow passage 114b extending from a distal end of the first axial flow passage 114a to an outer peripheral face 116 along the radial direction. Thus, the first fluid feeding passage 114 has a first opening 117 that opens on the outer peripheral face 116 of the sliding portion 112.

The second fluid feeding passage 115 has a second axial flow passage 115a extending from the proximal end of the cylindrical portion 113 along the axial direction, and a second radial flow passage 115b extending from a distal end of the second axial flow passage 115a to the outer peripheral face 116 along the radial direction. Thus, the second fluid feeding passage 115 has a second opening 118 that opens on the outer peripheral face 116 of the sliding portion 112. The second radial flow passage 115b is located on the proximal end side of the first radial flow passage 114b in the axial direction. The cylindrical portion 113 is configured to have an axial-direction length larger than the axial-direction length of the slide space 71e, and the first opening 117 and the second opening 118 of the cylindrical portion 113 is always located on the distal end side of the slide space 71e.

In a state that the proximal end side of the cylindrical portion 113 and the flange portion 72b are inserted into the slide space 71e, airtightness of an annular space formed by the outer peripheral portion 71d, the inner peripheral portion 71c, and a rear end of the sliding portion 112 is structurally maintained. In the sliding portion 112, the flange portion 72b is located in the slide space 71e, and slidably attached to the base portion 71. As illustrated in FIG. 10 (a) and 10 (b), the sliding portion 112 is configured such that, when the sliding portion 112 slides with respect to the base portion 71, the first opening 117 and the second opening 118 of the sliding portion 112 move to the proximal end side of the distal end of the distal end portion 71a and the distal end side of the distal end of the distal end portion 71a. Incidentally, since a material constituting the main body 110 is the same as the material constituting the main body 70 according to the seventh embodiment, the explanation in the seventh embodiment is applied to explanation of the material for the main body 110, and detailed explanation of the material for the main body 110 is omitted.

In the ligation device 901 according to the tenth embodiment, when searching for an affected part such as diverticula, the sliding portion 112 is sucked by the first tube 4a, so that the sliding portion 112 is located on the proximal end side, as illustrated in FIG. 10 (a). During ligation, the fluid is fed to the slide space 71e through the first tube 4a, so that the sliding portion 112 is moved to the distal end side, as illustrated in FIG. 10 (b). In this state, the fluid is delivered to the first fluid feeding passage 114 of the sliding portion 112 of the main body 110 through the second tube 4b, the first ligation ring 5a is ejected from the main body 110 using a pressure of the fluid as a driving source, and the affected part is ligated with the first ligation ring 5a. In this way, when searching for the affected part, the sliding portion 112 is located on the proximal end side, so that a wide visual field of the endoscope 2 can be maintained, and, during ligation, the sliding portion 112 is moved to the distal end side to allow the ligation.

Furthermore, after ligating one affected part with the first ligation ring 5a, the sliding portion 112 is sucked by the first tube 4a, so that the sliding portion 112 is located on the proximal end side, and another affected part is identified. The fluid is delivered to the second fluid feeding passage 115 of the sliding portion 112 of the main body 110 through the third tube 4d, and the second ligation ring 5b is ejected from the main body 110 using a pressure of the fluid as a driving source, and another affected part is ligated with the second ligation ring 5b. Thus, the ligation treatment can be continuously performed without taking out the ligation device 901 from the body.

### <Eleventh Embodiment>

The eleventh embodiment of the present invention will be explained with reference to the figures.

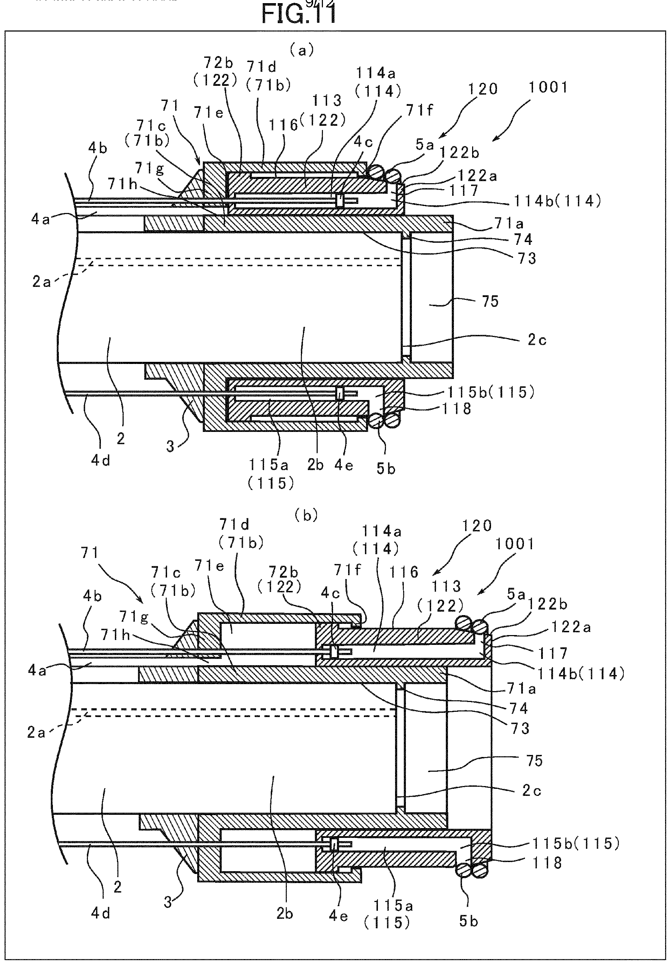

FIG. 11 (a) is a sectional view of a ligation device 1001 according to the eleventh embodiment of the present invention attached to the endoscope 2, illustrating a state that a sliding portion 122 is located on the proximal end side. FIG. 11 (b) is a sectional view of the ligation device 1001 attached to the endoscope 2, illustrating a state that the sliding portion 122 is located on the distal end side. FIG. 11 (a) and FIG. 11 (b) illustrate only the distal end portion of the endoscope 2 equipped with the ligation device 1001.

Additionally, in FIG. 11 (a) and FIG. 11 (b), the right side of the figure is a distal end side (farther side) where the device is to be inserted into a body, and the left side is a proximal end side (hand side, nearer side).

Note that, in the ligation device 1001 according to the eleventh embodiment, the same members as in the ligation device 601 according to the seventh embodiment are given the same reference numerals as in the seventh embodiment, and detailed explanation of the same members is omitted.

The ligation device 1001 includes the attachment cylinder 3, a main body 120, the first tube 4a, the second tube 4b, a third tube 4d, the first ligation ring 5a, and the second ligation ring 5b.

Since the main body 120 has the almost same configuration as of the main body 110 according to the tenth embodiment, the same components as in the tenth embodiment are given the same reference numerals as in the tenth embodiment, and only different components will be explained. A distal end portion 122a of the sliding portion 122 of the main body 120 has a tapered shape that tapers toward the distal end. In addition, the first opening 117 of the first fluid feeding passage 114 opens on an outer peripheral face 122b of the distal end portion 122a of the sliding portion 122, and the second opening 118 of the second fluid feeding passage 115 opens on the outer peripheral face 122b of the distal end portion 122a of the sliding portion 122.

The first ligation ring 5a is placed so as to airtightly obstruct the first opening 117 of the first fluid feeding passage 114. The second ligation ring 5b is placed so as to airtightly obstruct the second opening 118 of the second fluid feeding passage 115.

In the ligation device 1001 according to the eleventh embodiment, the first opening 117 and the second opening the first opening 118 open on the outer peripheral face 122b of the tapered distal end portion 122a, the first ligation ring 5a and the second ligation ring 5b are attached to the outer peripheral face 122b, and therefore the first ligation ring 5a and the second ligation ring 5b can be more reliably ejected from the main body 120.

### <Twelfth Embodiment>

The twelfth embodiment of the present invention will be explained with reference to the figures.

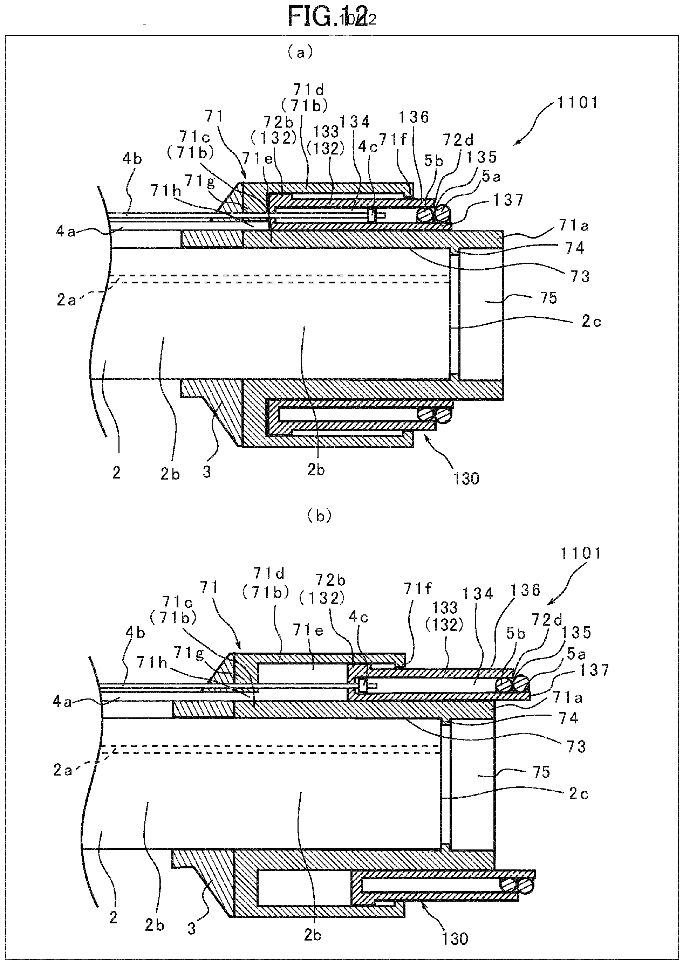

FIG. 12 (a) is a sectional view of a ligation device 1101 according to the twelfth embodiment of the present invention attached to the endoscope 2, illustrating a state that a sliding portion 132 is located on the proximal end side. FIG. 12 (b) is a front view of the ligation device 1101 attached to the endoscope 2, illustrating a state that the sliding portion 132 is located on the distal end side. FIG. 12 (a) illustrates only the distal end portion of the endoscope 2 equipped with the ligation device 1101.

Additionally, in FIG. 12 (a) and FIG. 12 (b), the right side of the figure is a distal end side (farther side) where the device is to be inserted into a body, and the left side is a proximal end side (hand side, nearer side).

Note that, in the ligation device 1201 according to the twelfth embodiment, the same members as in the ligation device 601 according to the seventh embodiment are given the same reference numerals as in the seventh embodiment, and detailed explanation of the same members is omitted.

The ligation device 1201 includes the attachment cylinder 3, a main body 130, the first tube 4a, the second tube 4b, the first ligation ring 5a, and the second ligation ring 5b.

The main body 130 has the cylindrical base portion 71 and the cylindrical sliding portion 132, and is connected to the distal end of the attachment cylinder 3, and located on the endoscope distal end portion 2b.

The sliding portion 132 has a cylindrical portion 133 and the flange portion 72b. The cylindrical portion 133 has a first fluid feeding passage 134 along the axial direction of the cylindrical portion 133. The first fluid feeding passage 134 is cylindrically formed coaxially with the cylindrical portion 133, and has a first annular opening 135 that opens on the distal end face 72d of the cylindrical portion 133 and is coaxial with the cylindrical portion 133. A distal end portion 136 of the cylindrical portion 133 has an annular protruding portion 137 located on the inner peripheral side of the first opening 135 and projecting toward the distal end side.

The first ligation ring 5a is attached to the outer periphery of the protruding portion 137 on the distal end portion 136 of the cylindrical portion 133 so as to airtightly obstruct the first annular opening 135. The second ligation ring 5b is attached to the proximal end side of the first ligation ring 5a on the first opening 136.

Since the ligation device 1101 according to the twelfth embodiment includes the second ligation ring 5b on the proximal end side of the first ligation ring 5a, the ligation treatment can be continuously performed without taking out the ligation device 1101 from the body.

### <Thirteenth Embodiment>

The thirteenth embodiment of the present invention will be explained with reference to the figures.

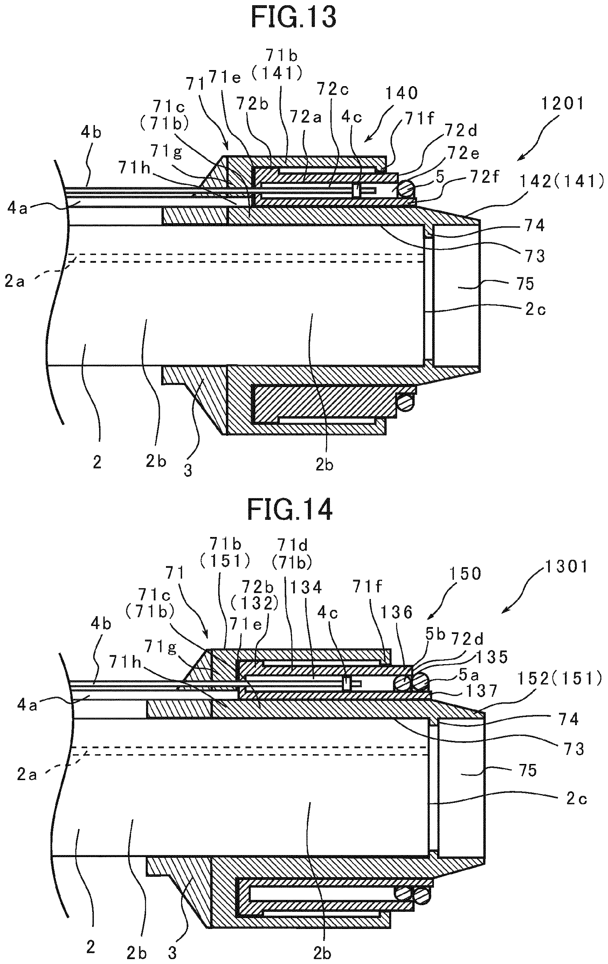

FIG. 13 is a sectional view of a ligation device 1201 according to the thirteenth embodiment of the present invention attached to the endoscope 2, illustrating a state that the sliding portion 72 is located on the proximal end side. FIG. 13 illustrates only the distal end portion of the endoscope 2 equipped with the ligation device 1201.

Additionally, in FIG. 13, the right side of the figure is a distal end side (farther side) where the device is to be inserted into a body, and the left side is a proximal end side (hand side, nearer side).

Note that, in the ligation device 1201 according to the thirteenth embodiment, the same members as in the ligation device 601 according to the seventh embodiment are given the same reference numerals as in the seventh embodiment, and detailed explanation of the same members is omitted.

The ligation device 1201 includes the attachment cylinder 3, a main body 140, the first tube 4a, the second tube 4b, and the first ligation ring 5.

The main body 140 has a cylindrical base portion 141 and the cylindrical sliding portion 72, and is connected to the distal end of the attachment cylinder 3, and located on the endoscope distal end portion 2b. The base portion 141 has a distal end portion 142 projecting toward the distal end side of the distal end face 2c of the endoscope 2, and the proximal end portion 71b. The distal end portion 142 has a tapered shape that tapers toward the distal end. In a state that the sliding portion 72 is located on the most proximal end side, the distal end of the cylindrical portion 72a of the sliding portion 72 is located on a proximal end of the distal end portion 142.

In the ligation device 1201 according to the thirteenth embodiment, when the first ligation ring 5 ejected from the sliding portion 72 moves to the tapered distal end portion 142, the first ligation ring 5 moves to the distal end side along the distal end portion 142 while a diameter of the first ligation ring 5 decreases without moving the sliding portion 72 to the distal end side of a distal end of the base portion 141, so that an affected part can be ligated.

<Fourteenth Embodiment>

The fourteenth embodiment of the present invention will be explained with reference to the figures.

FIG. 14 is a sectional view of a ligation device 1301 according to the fourteenth embodiment of the present invention attached to the endoscope 2, illustrating a state that the sliding portion 132 is located on the proximal end side. FIG. 14 illustrates only the distal end portion of the endoscope 2 equipped with the ligation device 1301.

Additionally, in FIG. 14, the right side of the figure is a distal end side (farther side) where the device is to be inserted into a body, and the left side is a proximal end side (hand side, nearer side).

Note that, in the ligation device 1301 according to the fourteenth embodiment, the same members as in the ligation device 1101 according to the twelfth embodiment are given the same reference numerals as in the twelfth embodiment, and detailed explanation of the same members is omitted.

The ligation device 1301 includes the attachment cylinder 3, a main body 150, the first tube 4a, the second tube 4b, the first ligation ring 5a, and the second ligation ring 5b.

The main body 150 has a cylindrical base portion 151 and the cylindrical sliding portion 132, and is connected to the distal end of the attachment cylinder 3, and located on the endoscope distal end portion 2b. The base portion 151 has a distal end portion 152 projecting toward the distal end side of the distal end face 2c of the endoscope 2, and the proximal end portion 71b. The distal end portion 152 has a tapered shape that tapers toward the distal end. In a state that the sliding portion 132 is located on the most proximal end side, the distal end of the cylindrical portion 133 of the sliding portion 132 is located on a proximal end of the distal end portion 152.

In the ligation device 1301 according to the fourteenth embodiment, when the first ligation ring 5a and second ligation ring 5b ejected from the sliding portion 132 move to the tapered distal end portion 152, the first and second ligation rings 5a and 5b move to the distal end side along the distal end portion 152 while diameters of the first and second ligation rings 5a and 5b decrease without moving the sliding portion 132 to the distal end side of a distal end of the base portion 151, so that an affected part can be ligated.

### <Fifteenth Embodiment>

The fifteenth embodiment of the present invention will be explained with reference to the figures.

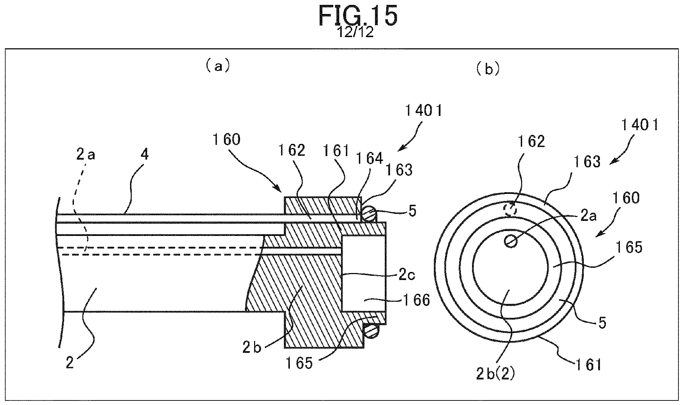

FIG. 15 is a partially cut-out sectional view of a ligation device 1401 according to the fifteenth embodiment of the present invention, including the endoscope portion 2. FIG. 15 illustrates only a distal end portion of the ligation device 1401 including an endoscope portion 2.

Additionally, in FIG. 15, the right side of the figure is a distal end side (farther side) where the device is to be inserted into a body, and the left side is a proximal end side (hand side, nearer side). Note that, in the ligation device 1401 according to the fifteenth embodiment, the same members as in the ligation device 1 according to the first embodiment are given the same reference numerals as in the first embodiment, and detailed explanation of the same members is omitted.

The ligation device 1401 includes a main body 160, the endoscope portion 2, the tube 4, and the first ligation ring 5. The endoscope portion 2 has the forceps hole 2a through which forceps not illustrated are inserted.

The main body 160 is cylindrical, located on an endoscope distal end portion 2b of the endoscope portion 2, and integrally attached to the endoscope distal end portion 2b. The main body 160 has a distal end portion 161 projecting toward the distal end side of the distal end face 2c of the endoscope portion 2. The main body 160 has a first fluid feeding passage 162 extending along the axial direction of the main body 160. The first fluid feeding passage 162 has a first opening 164 that opens on a distal end face 163 of the main body 160. The distal end portion 161 of the main body 160 is located on an inner peripheral side of the first opening 164 and has an annular protruding portion 165 projecting toward the distal end side. The distal end portion 161 and the distal end face 2c of the endoscope 2 constitute a recessed space 166. Incidentally, since a material constituting the main body 160 is the same as the material constituting the main body 10 according to the first embodiment, the explanation in the first embodiment is applied to explanation of the material for the main body 160, and detailed explanation of the material for the main body 160 is omitted.

The tube 4 extends from the distal end portion to the proximal end portion along the endoscope portion 2. The distal end of the tube 4 is airtightly connected to a proximal end of the first fluid feeding passage 162.

The first ligation ring 5 is attached to an outer periphery of the protruding portion 165 on the distal end portion 161 of the main body 160 so as to airtightly obstruct the first opening 164.

In the ligation device 1401 according to the fifteenth embodiment, the first ligation ring 5 is attached to the distal end portion 161 of the main body 160 so as to airtightly obstruct the first opening 164. Thereby, the first ligation ring 5 can be reliably ejected from the main body 140 such that the first ligation ring 5 is pushed out using a pressure of a fluid flowing through the first fluid feeding passage 162 as a driving source.

Although the embodiments of the present invention have been described above, the present invention is not limited to the aforementioned embodiments and can be variously modified.

For example, in the above embodiments, the fluid for ejecting the ligation rings 5, 5a and 5b to drive the sliding portions 72, 82, 92, 112, 122 and 132 is air, but the fluid may be a liquid such as physiological saline. Although the ligation devices according to the above embodiments are configured such that one or two ligation rings are ejected, it is possible to adopt a configuration that three or more ligation rings can be ejected. Incidentally, a ring-shaped member made of a shape-memory alloy can be used as the ligation ring, for example. A shape of such a ring-shaped member changes from circle to non-circle when ejected from the main body of the ligation device, and therefore ligation can be performed according to a shape of an affected part. In addition, as the ligation ring, a clip made of a shape-memory alloy can be used. When attached to the main body of the ligation device, such a clip is ring-shaped, and when ejected from the main body, the clip is transformed into a previously-memorized shape.

### DESCRIPTION OF REFERENCE NUMERALS

1, 101, 201, 301, 401, 501, 601, 701, 801, 901, 1001, 1101, 1201, 1301, 1401 Ligation device
5, 5a First ligation ring
5b Second ligation ring
10, 20, 30, 40, 50, 60, 70, 80, 90, 110, 120, 130, 140, 150, 160 Main body
11, 21, 31, 41, 51, 61, 71a, 142, 152, 161 Distal end portion
12, 22, 34a, 42, 62, 72c, 82b, 92c, 114, 134, 162 First fluid feeding passage
13, 32, 63 Distal end face of distal end portion
14, 24, 35a, 45a, 64, 72e, 82f, 92g, 117, 135, 164 First opening
23, 44, 54 Outer peripheral face of distal end portion
34b, 43, 92f, 115 Second fluid feeding passage
35b, 45b, 92h, 118 Second opening
71, 141, 151 Base portion
72, 82, 92, 112, 122, 132 Sliding portion
72d, 92b Distal end face of sliding portion
82d, 116, 122B: Outer peripheral face of sliding portion

## Claims

1. A ligation device comprising:
a main body having a cylindrical shape, located on an endoscope distal end portion, having a distal end portion projecting toward a distal end side of a distal end face of the endoscope, and having a first fluid feeding passage with a first opening that opens on a distal end face or an outer peripheral face of the distal end portion; and
a first ligation ring attached to the distal end portion of the main body so as to obstruct the first opening.

2. The ligation device according to claim 1, wherein
the main body has a second fluid feeding passage with a second opening that opens on the distal end face or the outer peripheral face of the distal end portion, and
a second ligation ring is attached to the distal end portion of the main body so as to obstruct the second opening of the second fluid feeding passage.

3. The ligation device according to claim 2, wherein
the distal end portion of the main body has a tapered shape that tapers toward the distal end, and the first opening of the first fluid feeding passage and the second opening of the second fluid feeding passage open on the outer peripheral face of the distal end portion of the main body.

4. The ligation device according to claim 1, wherein
the main body comprises
a cylindrical base portion and
a cylindrical sliding portion placed so as to be slidable with respect to the base portion by a fluid,
the first fluid feeding passage is formed on the sliding portion, the first opening opens on a distal end face or an outer peripheral face of the sliding portion, and
the first ligation ring is attached to the sliding portion.

5. The ligation device according to claim 4, wherein
a distal end portion of the base portion has a tapered shape that tapers toward the distal end.

6. The ligation device according to claim 5, wherein
the sliding portion has a second fluid feeding passage having a second opening that opens on the distal end face or the outer peripheral face of the sliding portion, and
a second ligation ring is attached to the sliding portion so as to obstruct the second opening of the second fluid feeding passage.

7. The ligation device according to claim 6, wherein
a distal end portion of the sliding portion has a tapered shape that tapers toward the distal end, and the first opening of the first fluid feeding passage and the second opening of the second fluid feeding passage open on an outer peripheral face of the distal end portion of the sliding portion.

8. The ligation device according to claim 1, wherein
the first opening has an annular shape coaxial with the cylindrical main body,
the first ligation ring is placed on the main body so as to obstruct the first annular opening, and
a second ligation ring is placed on a proximal end side of the first opening with respect to the first ligation ring.

9. The ligation device according to claim 1, wherein
the main body comprises
a cylindrical base portion and
a cylindrical sliding portion placed so as to be slidable with respect to the base portion by a fluid,
the first fluid feeding passage is formed on the sliding portion, the first opening opens on a distal end face of the sliding portion, and
the first opening has an annular shape coaxial with the cylindrical sliding portion.

10. The ligation device according to claim 9, wherein
a distal end portion of the base portion has a tapered shape that tapers toward the distal end.

11. The ligation device according to any one of claims 1 to 10, wherein
a projection on which a distal end of the endoscope distal end portion abuts is placed on an inner peripheral face of the main body.

12. The ligation device according to any one of claims 1 to 10, wherein
the main body is made of a translucent material.
